# EUROPÄISCHE PATENTSCHRIFT

(11) **EP 1 408 984 B1**
(45) Veröffentlichungstag und Bekanntmachung des Hinweises auf die Patenterteilung: **22.10.2008**
(21) Anmeldenummer: 02776913.2
(22) Anmeldetag: 18.07.2002
(51) Int. Cl.: A61K 31/66, C07F 9/06, C07F 9/28, C07F 9/655

(54) **ORGANO-PHOSPHORVERBINDUNGEN ZUR AKTIVIERUNG VON GAMMA/DELTA-T-ZELLEN**
ORGANO-PHOSPHOROUS COMPOUNDS FOR ACTIVATING GAMMA/DELTA T CELLS
COMPOSES ORGANOPHOSPHORES POUR L'ACTIVATION DE CELLULES T GAMMA/DELTA

(30) Priorität: 20.07.2001 DE 10134705; 25.07.2001 DE 10135395
(43) Veröffentlichungstag der Anmeldung: 21.04.2004
(73) Patentinhaber: BioAgency AG, 22525 Hamburg (DE)
(72) Erfinder: JOMAA, Hassan, 35392 Giessen (DE); WOLF, Oliver, 61197 Florstadt (DE); ALTINCICEK, Boran, 35463 Fernwald-Annerod (DE); EBERL, Mathias, 35390 Giessen (DE); HINTZ, Martin, 35398 Giessen (DE); KOLLAS, Ann-Kristin, 35390 Giessen (DE); REICHENBERG, Armin, 35510 Butzbach (DE); WIESNER, Jochen, 35394 Giessen (DE)
(74) Vertreter: Becker, Philippe
(86) Internationale Anmeldenummer: PCT/EP2002/007986
(87) Internationale Veröffentlichungsnummer: WO 2003/009855

(56) Entgegenhaltungen:
- WO-A-00/30653
- WO-A-00/59916
- WO-A-02/083720
- BELMANT, CHRISTIAN ET AL: "3-Formyl-1-butyl pyrophosphate a novel mycobacterial metabolite-activatin human.gamma..delta. T cells" JOURNAL OF BIOLOGICAL CHEMISTRY (1999), 274(45), 32079-32084, XP002225541 in der Anmeldung erwähnt
- TAKAGI ET AL.: TETRAHEDRON LETTERS, Bd. 41, 2000, Seiten 3395-3398, XP002225542
- TANAKA Y ET AL: "Natural and synthetic non-peptide antigens recognized by human.gamma..delta. T cells" NATURE, MACMILLAN JOURNALS LTD. LONDON, GB, Bd. 375, 11. Mai 1995 (1995-05-11), Seiten 155-158, XP002102418 ISSN: 0028-0836
- MCCLARD, R.W.; ET AL.: J. AM. CHEM. SOC., Bd. 109, 1987, Seiten 5544-5545, XP002231961
- JACOB L ET AL: "ON THE INFLUENCE OF PHOSPHORIC ESTER GROUPS IN GERANYLDIPHOSPHATE BIOSYNTHESIS" BULLETIN DE LA SOCIETE CHIMIQUE DE FRANCE, XX, XX, Bd. 127, Nr. 6, 1990, Seiten 719-733, XP009003286
- SIRECI, G.; ET AL.: EUR. J. IMMUNOL., Bd. 31, 2001, Seiten 1628-1635, XP002231962
- BELMANT, C.; ET AL.: THE FASEB JOURNAL, Bd. 14, 2000, Seiten 1669-1670, XP002231963
- HINTZ, M.; ET AL.: FEBS LETTERS, Bd. 509, 2001, Seiten 317-322, XP002231964
- FEURLE J.; ET AL.: THE JOURNAL OF BIOLOGICAL CHEMISTRY, Bd. 277, Nr. 1, 2002, Seiten 148-154, XP002231965
- ESPINOSA, E.; : THE JOURNAL OF IMMUNOLOGY, Bd. 168, 2002, Seiten 6336-6343, XP002231966
- GAO, W.; ET AL.: ANGEW. CHEM. INT. ED, Bd. 41, Nr. 14, 2002, Seiten 2604-2607, XP002231967
- SEEMANN, M.; ET AL.: TETRAHEDRON LETTERS, Bd. 43, 2002, Seiten 1413-1415, XP002231968
- WARD, J.L.; ET AL.: J. CHEM. SOC., PERKIN TRANS. 1, 2002, Seiten 710-712, XP002231969
- FOX, D.T.; ET AL.: J. ORG. CHEM., Bd. 67, 2002, Seiten 5009-5010, XP002231970

## Beschreibung

Zahlreiche Erkrankungen bei Mensch und Tier sind auf Fehlfunktionen des Immunsystems zurückzuführen. Es besteht daher ein hoher Bedarf an Substanzen, die in der Lage sind, das Immunsystem zu regulieren.

Die Biosynthese von Isoprenoiden über den klassischen Acetat / Mevalonat-Weg (Beytia ED, Porter JW. Annu Rev Biochem. 1976;45:113-42) und einen alternativen, Mevalonatunabhängigen Biosyntheseweg, den 2 Methyl-D-erythritol-Weg (MEP-Weg, synonym DOXP-Weg) ist bekannt (Rohmer M. Nat Prod Rep. 1999 Oct;16(5):565-74). Beide Wege führen zu Isopentenylpyrophosphat (IPP), dem gemeinsamen Vorläufer aller höheren Isoprenoide. Während der Acetat / Mevalonat-Weg seit längerem bekannt und vollständig aufgeklärt ist, sind derzeit noch nicht alle biosynthetischen Reaktionsschritte des MEP-Wegs bekannt.

Es ist bekannt, daß humane gamma / delta-T-Zellen durch ein oder mehrere Intermediate des MEP-Wegs aktiviert werden. Dies bedeutet, daß es bei Inkubation von peripheren Blutlymphozyten mit Extrakten aus Organismen, die den MEP-Weg besitzen, zu einer selektiven Prolliferation und Zytokinsekretion der gamma / delta-T-Zellpopulation kommt (Jomaa H, Feurle J, Luhs K, Kunzmann V, Tony HP, Herderich M, Wilhelm M. FEMS Immunol Med Microbiol. 1999 Sep;25(4):371-8). Die genaue chemisch Beschaffenheit dieser aktivierenden Substanz oder Substanzen ist noch unbekannt. Publizierte Daten sprechen dafür, daß 3-Pormyl-1-Butylpyrophosphat als hypothetisches Intermediat des MEP-Wegs ebenso wie Isopentenyl-Pyrophosphat eine Rolle bei der Aktivierung von gamma / delta-T-Zellen spielt (Belmant C, Espinosa E, Poupot R, Peyrat MA, Guiraud M, Poquet Y, Bonneville M, Fournie JJ. J Biol Chem. 1999 Nov 5;274(45):32079-84, Tanaka, Y, et al," National and synthetic non-peptide antigens recognized by human gamma-delta Tcells", Nature, May 1995, 375, 155-158; Belman C et al, The FASEB Journal, 2000, 14, 1669-1670; Sireci et al, Eur J. Immunol, 2001, 31, 1628-1635). Andere Derivaten als einfachen Prenylderivaten sind also bekannt (Mc Land, Rw, et al, Ann. Chem. Soc, 1987, 109, 5544-5545, Jacot L et al, Buffet in de la Société Chimique de France, Vol 127, No 6, 1990, 719-739)

Aufgabe dieser Erfindung ist es Substanzen zur Verfügung zu stellen, die in der Lage sind gamma / delta-T-Zellen zu stimulieren und damit regulierend auf das Immunsystem einzuwirken.

Diese Aufgabe wird durch Arzneimittel, die eine oder mehrere der in Anspruch 1 sowie den Unteransprüchen definierten Substanzen enthalten, gelöst.

Überraschender Weise hat sich gezeigt, daß Verbindungen gemäß Formel (I) hervorragend zur Aktivierung von gamma / delta T-Zellen geeignet sind. 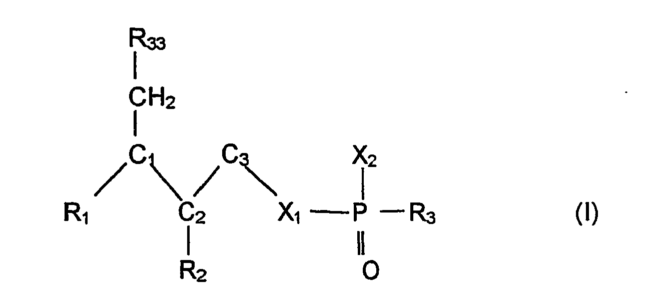 wobei R₁ aus der Gruppe ausgewählt ist, die aus einem Methyl-, einem Formylrest, substituierten und unsubstituierten Hydroxymethylresten und C₀H₂R₃₁ besteht, wobei R₃₁ aus der Gruppe ausgewählt ist, die aus OH, substituiertem und unsubstituiertem Phosphat und substituiertem und unsubstituiertem Pyrophosphat besteht und R₃₁ und R₂ nicht gleichzeitig im Molekül vorhanden sein können,
R₃₃ aus der Gruppe ausgewählt ist, die aus Wasserstoff, OH, substituiertem und unsubstituiertem Phosphat und substituiertem und unsubstituiertem Pyrophosphat besteht,
R₃ aus der Gruppe ausgewählt ist, die aus Wasserstoff, substituiertem und unsubstituiertem Alkyl mit 1 bis 26 Kohlenstoffatomen, substituiertem und unsubstituiertem Hydroxyalkyl mit 1 bis 26 Kohlenstoffatomen, substituiertem und unsubstituiertem Aryl, substituiertem und unsubstituiertem Aralkyl, substituiertem und unsubstituiertem Alkenyl mit 1 bis 26 Kohlenstoffatomen, substituiertem und unsubstituiertem Alkinyl mit 1 bis 26 Kohlenstoffatomen, substituiertem und unsubstituiertem Cycloalkyl, substituiertem und unsubstituiertem heterocyclischem Rest, substituiertem oder unsubstituiertem Phosphat, einem Silyl, einem Nucleosid, einem Nucleosidmono-, -di- oder -triphosphat, einem Desoxynucleosid, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, und OR₃₄ besteht, wobei R₃₄ wie R₃ definiert ist,
X₂, sofern zwischen X₂ und C₁ ein Cyclus gebildet wird, wie X₁ definiert ist und ansonsten X₂ aus der Gruppe ausgewählt ist, die aus -OR₆, 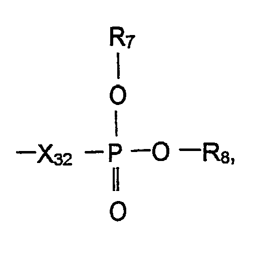 , wobei R₇ und R₈ definiert sind wie R₃₄. 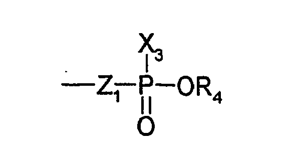 besteht, wobei R₄ wie R₃ und Z₁ wie X₁ definiert sind und X₃, wenn es mit C₁ einen Cyclus bildet, wie X₁ definiert ist und, wenn es keinen Cyclus mit C₁ bildet, einer

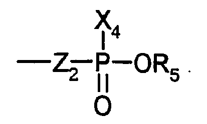

Gruppe entspricht, wobei R₅ wie R₃ definiert ist und Z₂ und X₄, das mit C₁ einen Cyclus bildet, wie X₁ definiert sind.
R₂ aus der Gruppe ausgewählt ist, die aus Wasserstoff, OH, Alkoxy, Phenoxy, Benzyloxy, substituiertem und unsubstituiertem Phosphat und substituiertem und unsubstituiertem Pyrophosphat besteht,
X₁ Sauerstoff oder 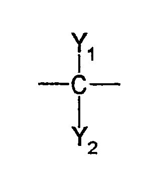 sein kann, wobei Y₁ und Y₂, die gleich oder verschieden sein können, ausgewählt sind aus der Gruppe, die H, OH, Halogen, eine Amino-, eine C₁₋₉-Alkoxy und eine C₁₋₉-AlkylthioRest umfasst, oder gemeinsam eine Oxogruppe bilden,
und zwischen C₀ und C₁ oder C₁ und C₂ oder C₂ und C₃ eine Doppelbindung vorliegen kann.

Bevorzugt sind die Verbindungen der Formel: 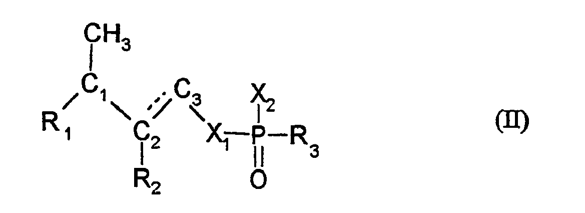 wobei zwischen C₂ und C₃ eine Einfach- oder eine Doppelbindung vorhanden ist, R₁ aus der Gruppe ausgewählt ist, die aus einem Methyl-, einem Formyl-Rest und substituierten oder unsubstituierten Hydroxymethyl-Resten besteht,
R₂ aus der Gruppe ausgewählt ist, die aus Wasserstoff, Hydroxy-, Alkoxy-, Phenoxy-, Benzyloxy-Resten, substituiertem oder unsubstituiertem Phosphat und substituiertem oder unsubstituiertem Pyrophosphat besteht,
X₁ Sauerstoff ist oder einer

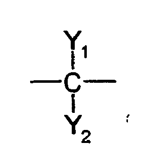

Gruppe entspricht, wobei Y₁ und Y₂, die gleich oder verschieden sein können, ausgewählt sind aus der Gruppe, die H, OH, Halogen, Amino, C₁₋₉-Alkoxy- und C₁₋₉-AlkylthioReste umfasst, oder gemeinsam eine Oxogruppe bilden,

R₃ ausgewählt ist aus der Gruppe, die aus Wasserstoff, substituiertem und unsubstituiertem Alkyl mit 1 bis 26 Kohlenstoffatomen, substituiertem und unsubstituiertem Hydroxyalkyl mit 1 bis 26 Kohlenstoffatomen, substituiertem und unsubstituiertem Aryl, substituiertem und unsubstituiertem Aralkyl, substituiertem und unsubstituiertem Alkenyl mit 1 bis 26 Kohlenstoffatomen, substituiertem und unsubstituiertem Alkinyl mit 1 bis 26 Kohlenstoffatomen, substituiertem und unsubstituiertem Cycloalkyl, substituiertem und unsubstituiertem heterocyclischem Rest, substituiertem oder unsubstituiertem Phosphat, einem Silyl, einem Nucleosid, einem Nucleosidmono-, -di- oder Triphosphat, einem Desoxynucleosid, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht,
X₂, sofern zwischen X₂ und C₁ ein Cyclus gebildet wird, wie X₁ definiert ist und ansonsten X₂ 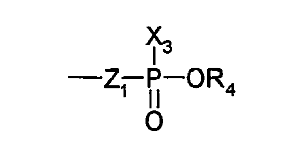 entspricht, wobei R₄ wie R₃ und Z₁ wie X₁ definiert sind und X₃, wenn es mit C₁ einen Cyclus bildet, wie X₁ definiert ist und, wenn es keinen Cyclus mit C₁ bildet, einer

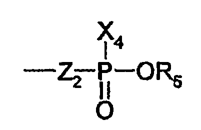

Gruppe entspricht, wobei R₅ wie R₃ definiert ist und Z₂ und X₄, das mit C₁ einen Cyclus bildet, wie X₁ definiert sind.

Ferner sind Verbindungen bevorzugt, die der folgenden Formel entsprechen: 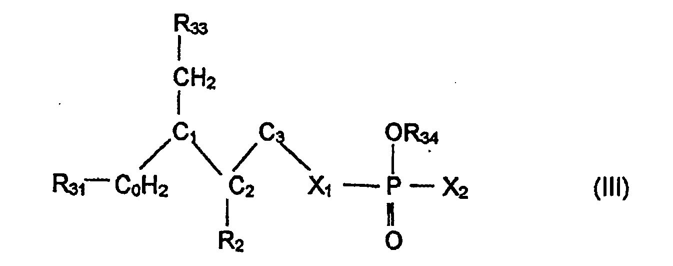 R₃₁ und R₂, die nicht gleichzeitig im Molekül vorhanden sein können, sind ausgewählt aus der Gruppe, die aus OH, substituiertem und unsubstituiertem Phosphat und substituiertem und unsubstituiertem Pyrophosphat besteht, ist R₃₁ im Molekül vorhanden, so wird zwischen C₁ und C₂ eine Doppelbindung gebildet, analog bildet sich zwischen C₀ und C₁ eine Doppelbindung aus, wenn R₂ im Molekül vorhanden ist, R₃₃ ist ausgewählt aus der Gruppe, die aus Wasserstoff, OH, substituiertem und unsubstituiertem Phosphat und substituiertem und unsubstituiertem Pyrophosphat besteht, R₃₄ ist ausgewählt aus der Gruppe die aus Wasserstoff, substituiertem und unsubstituiertem Alkyl mit 1 bis 26 Kohlenstoffatomen, substituiertem und unsubstituiertem Hydroxyalkyl mit 1 bis 26 Kohlenstoffatomen, substituiertem und unsubstituiertem Aryl, substituiertem und unsubstituiertem Aralkyl, substituiertem und unsubstituiertem Alkenyl mit 1 bis 26 Kohlenstoffatomen, substituiertem und unsubstituiertem Alkinyl mit 1 bis 26 Kohlenstoffatomen, substituiertem und unsubstituiertem Cycloalkyl, substituiertem und unsubstituiertem heterocyclischem Rest, substituiertem oder unsubstituiertem Phosphat, einem Silyl, einem Nucleosid, einem Desoxynucleosid, einem Nucleosidmono-, -di- oder triphosphat, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht, X₂ ist entweder -OR₆, wobei R₆ analog R₃₄ definiert ist oder kann 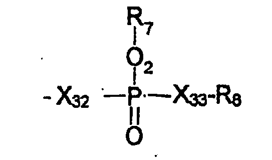 sein, wobei R₇ und R₈ definiert sind wie R₃₄.
X₁, X₃₂ und X₃₃, die gleich oder verschieden sein können, können Sauerstoff oder eine 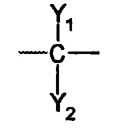 Gruppe, wobei Y₁ und Y₂, die gleich oder verschieden sein können, ausgewählt sind aus der Gruppe, die H, OH, Halogen, eine Amino-, eine C₁₋₉-Alkoxy und eine C₁₋₉-Alkylthio-Rest umfasst, oder gemeinsam eine Oxogruppe bilden, wobei wenn R₂ ist abwesend, R₃₃ ist H und R₃₁ ist OH, dann das Segment X₁-PO(OR₃₄)-X₂ ist nicht -O-PO(OH)-OPO(OH)₂ oder -O-PO(ON)₂

Besonders bevorzugt sind Verbindungen gemäß Formel (IIIA) 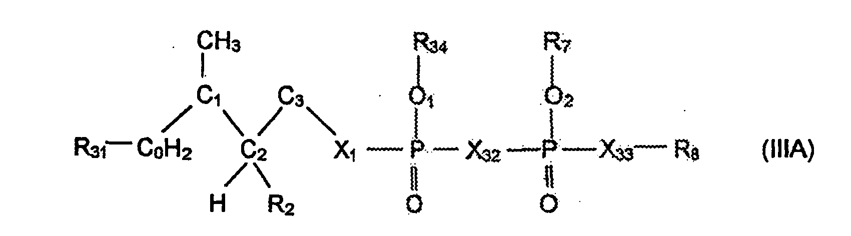 wobei R₃₁ und R₂, die nicht gleichzeitig im Molekül vorhanden sein können, ausgewählt sind aus der Gruppe, die aus OH, substituiertem und unsubstituiertem Phosphat und substituiertem und unsubstituiertem Pyrophosphat besteht, ist R₃₁ im Molekül vorhanden, so wird zwischen C₁ und C₃ eine Doppelbindung gebildet, analog bildet sich zwischen C₀ und C₁ eine Doppelbindung aus, wenn R₂ im Molekül vorhanden ist, R₃₄, R₇ und R₈, die gleich oder verschieden sein können sind definiert wie oben,
X₁, X₃₂ und X₃₃ die gleich oder verschieden sein können, sind definiert wie bei Verbindung (III).

In den Formeln (I) bis (IIIA) sind aus Gründen der Übersichtlichkeit Wasserstoffsubstuenten an C₁, C₂ und C₃ nicht explizit angegeben. Es versteht sich jedoch, daß C-Atome vierbindig sind. Die fehlenden Substituenten sind folglich Wasserstoffreste.

Bevorzugt sind ferner Verbindungen gemäß Formel (IIIA), bei denen R₃₁ bzw. R₂ entweder OH oder ein substituiertes oder unsubstituiertes Phosphat, R₃₄, R₇ und R₈ ausgewählt aus der Gruppe, die aus substituiertem und unsubstituiertem Phosphat, einem Nucleosid, einem Desoxynucleosid, einem Nucleosidmono-, -di- oder triphosphat, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht und X₁, X₃₂ und X₃₃, die gleich oder verschieden sein können, gleich O, CHF, CHCl, CFCl, CH₂, CF₂, oder CCl₂ sind.

Bevorzugt sind weiterhin Verbindungen gemäß Formel (IIIA), bei denen die Phosphat Gruppen als Natrium-, Kalium- oder substituierte bzw. unsubstituierte Ammoniumsalze vorliegen.

Am Besten geeignet sind nachfolgende Verbindungen:

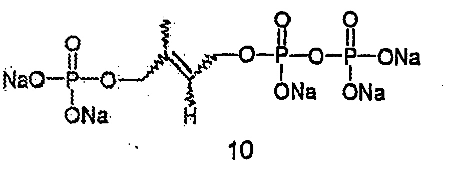

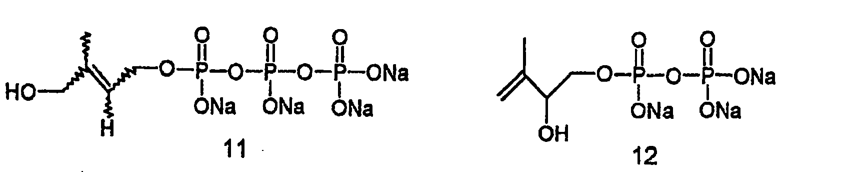

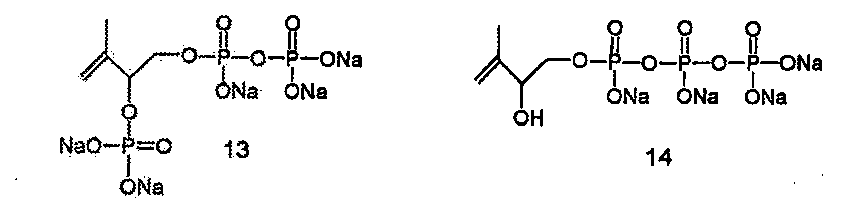

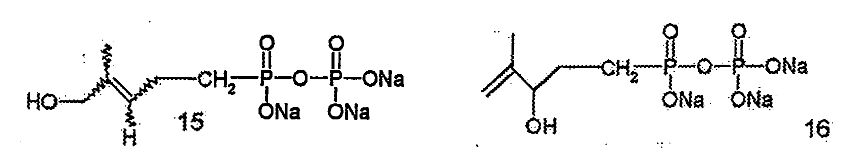

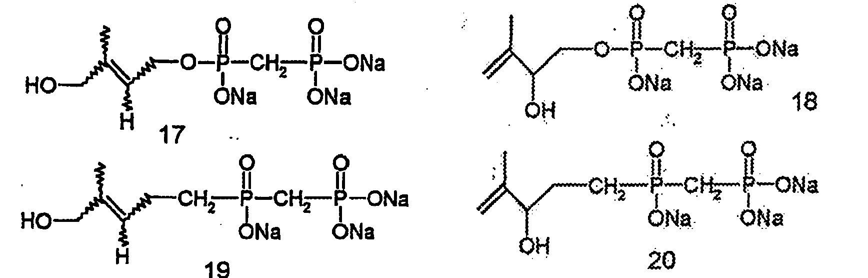

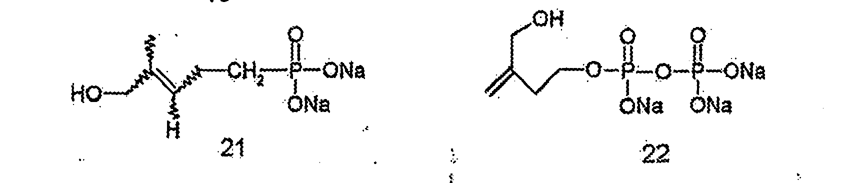

Andere Weiterbildungen der Erfindung sind durch, die Unteransprüche definiert.

Besonderheiten der obigen Definitionen und geeignete Beispiele dafür werden nachfolgend angegeben:

"Alkyl" ist ein gerad- oder verzweigtkettiger Alkylrest mit bis zu 26 Kohlenstoffatomen, soweit nicht anders definiert, wie Methyl, Ethyl, Propyl, Isopropyl, Butyl, Isobutyl, tert.-Butyl, Pentyl, Hexyl und dergleichen.

Zu "Alkenyl" gehören gerad- oder verzweigtkettige Alkenylgruppen mit bis zu 26 Kohlenstoffatomen, soweit nicht anders definiert, wie z.B. Vinyl, Propenyl (z.B. 1-Propenyl, 2-Propenyl), 1-Methylpropenyl, 2-Methylpropenyl, Butenyl, 2-Ethylpropenyl, Pentenyl, Hexenyl.

Zu "Alkinyl" gehören gerad- oder verzweigtkettige Alkinylgruppen mit bis zu 26 Kohlenstoffatomen, soweit nicht anders definiert.

Cycloalkyl steht vorzugsweise für ein ggfs. substituiertes C₃-C₇-Cycloalkyl; als mögliche Substituenten sind u.a. Alkyl, Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl, Naphthyl usw., der ggf. einen oder mehrere geeignete Substituenten aufweisen kann, wie Alkoxy (z.B. Methoxy, Ethoxy etc.), Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. einen oder mehrere geeignete Substituenten aufweisen kann, wie Alkoxy (z.B. Methoxy, Ethoxy etc.), Halogen (z.B. Fluor, Chlor, Brom etc.), Nitro und dergleichen.

"Alkoxyrest" ist, soweit nicht anders definiert, ein gerad- oder verzweigtkettiger Alkoxyrest mit bis zu 26 Kohlenstoffatomen, wie ein Methoxy, Ethoxyreste, etc.. Er kann z.B. mit Hydroxy-, Amino-, Halogen-, Oxogruppen und Alkoxyresten, wie Methoxy-, Ethoxyresten, substituiert sein.

"Hydroxymethylrest" ist, soweit nicht anders definiert, ein Rest, bei dem am Sauerstoff ein substituierter oder unsubstituierter C₁-C₉-Alkyl-, Aryl- oder Aralkylrest, wie z.B. Methoxymethyl, Ethoxymethyl, Phenoxymethyl oder Benzyloxymethyl usw. hängt oder bei dem am Sauerstoff ein substituierter bzw. unsubstituierter Phosphat- oder Pyrophosphatrest, wie z.B. Adenosindiphosphat, Uridindiphosphat usw., hängt.

"Alkylthiorest" ist, soweit nicht anders definiert, ein gerad- oder verzweigtkettiger Alkylthiorest mit bis zu 9 Kohlenstoffatomen, wie ein Thiomethyl-, Thioethylreste etc. Er kann z.B. mit Hydroxy-, Amino-, Halogen-, Oxogruppen und Alkoxyresten, wie Methoxy-, Ethoxyresten, substituiert sein.

"Silylreste" können zum Beispiel mit den wie oben definierten Alkylresten oder Cycloalkyl-(C₀₋₂₆)-alkylresten substituiert sein.

"Silyl"-(C₀₋₂₆)-alkylgruppen" sind Silykeste, die auch über einen Alkylrest an das Grundgerüst gebunden sein können. Die Alkyl- und Silylgruppen sind wie oben definiert.

Bei den obigen Estern kann der Alkan- und/oder Arenteil wahlweise zumindest einen geeigneten Substituenten aufweisen, wie Halogen, Alkoxy, Hydroxy, Nitro oder dergleichen.

Zu substituierten und unsubstituierten Phosphatresten bzw. substituierten und unsubstituierten Pyrophosphatresten gehören Salzverbindungen der entsprechenden Phosphorsäurederivate mit organischen oder anorganischen Basen (z.B. Natriumsalz, Kaliumsalz, Calciumsalz, Aluminiumsalz, Ammoniumsalz, Magnesiumsalz, Triethylaminsalz, Ethanolaminsalz, Dicyclohexylaminsalz, Ethylendiaminsalz, N,N'-Dibenzylethylendiaminsalz etc.) sowie Salze mit Aminosäuren (z.B. Argininsalz, Lysinsalz, Glycinsalz, Alaninsalz, Ornithinsalz, etc.), sowie Reste bei denen die Phosphatgruppe Ester mit substituiertem oder unsubstituiertem C₁-C₂₆-Alkyl-, substituiertem oder unsubstituiertem Aryl, substituiertem oder unsubstituiertem Aralkyl, substituiertem oder unsubstituiertem Cycloalkyl oder substituiertem oder unsubstituiertem Heterocyclischen Rest, oder einem Nucleosid oder einem Desoxynucleosid, bildet.

Unter "Nucleosid" sind Adenosin, Guanosin, Uridin, Thymidin und Cytidin, unter "Desoxynucleosid" sind Desoxyadenosin, Desoxyguanosin, Desoxythymidin, Desoxycytidin und Desoxyuridin zu verstehen.

Die Erfindung beinhaltet ebenfalls die pharmazeutischen Salze und Ester der Salze. Ferner sind alle räumlichen Isomere der Verbindungen, sowohl als Reinstoffe als auch in Form ihrer Mischungen beinhaltet.

Die erfindungsgemäßen Substanzen könne aus Bakterien, Algen, Pflanzen und Protozoen, auch solchen, bei denen das lytB-Gen deletiert worden ist, gewonnen und aufgereinigt werden (Beispiel 1). Die Aufreinigung kann mittels HPLC oder durch andere an sich bekannte Methoden wie Elektrophorese, Fällung (z.B. als Bariumsalz) oder andere chromatographische Verfahren erfolgen.

Es sind verschiedene Anwendungen der Verbindungen möglich. So hat sich z.B. gezeigt, dass die Substanzen für Aktivitätstests der Enzyme GcpE und LytB, sowie in Testsystemen zur Messung der Aktivierung von gamma / delta-T-Zellen (siehe Beispiel 2, 5) verwendet werden können.

Die erfindungsgemäßen Substanzen können entweder chemisch synthetisiert (Beispiel 3) oder aus Bakterien, Algen, Pflanzen und Protozoen, gewonnen und aufgereinigt werden(Beispiel 4). Die Aufreinigung kann mittels HPLC oder durch andere an sich bekannte Methoden wie Elektrophorese, Fällung (z.B. als Bariumsalz) oder andere chromatographische Verfahren erfolgen.

Ferner können die erfindungsgemäßen Substanzen, da sie Intermediate des MEP-Wegs darstellen, in einem Screening-Verfahren zur Identifikation von Inhibitoren des GcpE- und des LytB-Enzyms eingesetzt werden. Dieses Verfahren zur Aktivitätsbestimmung der Enzyme beruht auf der Messung von Konzentrationsunterschieden der Substrate und Produkte der Enzyme unter geeigneten Reaktionsbedingungen. Durch In-Kontakt-Bringen geeigneter Testsubstanzen mit den Enzymen während der Aktivitätsbestimmung können Inhibitoren durch Verringerung der beobachteten Enzymaktivität identifiziert werden. Die Inhibitoren eignen sich als Herbizide und als Wirkstoffe mit antibakterieller, antiparasitärer und antiviraler Aktivität bei Mensch und Tier.

Die erfindungsgemäßen Verbindungen können auch zur Herstellung von Arzneimitteln verwendet werden. Die Wirksamkeit der Verbindungen beruht dabei auf der Aktivierung von gamma / delta-T-Zellen. Abhängig vom Anwendungsbereich kann dadurch die Immunabwehr gestärkt werden oder eine immunologische Toleranz gegen Autoantigene und Allergene induziert werden.

Anwendungsbereiche sind die Behandlung von Immun-, Autoimmunerkrankungen und Allergien bei Mensch und Tier. Beispiele hierfür sind: Allergien, Multiple Sklerose, Rheumatoide Arthritis, Hashimoto-Thyreoiditis, Myasthenia Gravis, Lupus Erythematodes, Diabetes Mellitus, primär-biliäre Zirrhose, aktive chronische Hepatitis, Adrenalinitis/Addison-Krankheit, Polymyositis, Dermatomyositis, Autoimmunhämolytische Anämie, Herzmuskel- und Herzhautentzündungen, Sklerodermie, Uveitis (Phakouveitis, sympathische Opthalmie), Pemphigus Vulgaris, Pemphigoid, Perniziöse Anämie, Autoimmune atrophische Gastritis, entzündliche Erkrankungen des Darms wie Crohn-Krankheiten und Colitis Ulcerosa, entzündliche Erkrankungen der Lunge wie Asthmatische Erkrankungen und Bronchitiden.

Bevorzugt ist die Anwendung bei Morbus Crohn, Colitis Ulcerosa, Multipler Sklerose, Asthma, chronische Bronchitis, Allergien.

Es zeigte sich ferner, das die erfindungsgemäßen Substanzen erfolgreich zur Behandlung von Erkrankungen, verursacht durch Viren, Bakterien und Parasiten, eingesetzt werden können.

Insbesondere eignen sich die durch Anspruch 1 sowie die Unteransprüche definierten Substanzen zur Vorbeugung und Behandlung von Tumoren, die durch Mikroorganismen verursacht werden. Zu dieser Gruppe von Mikroorganismen gehören Bakterien, wie Helicobacter pylori (z.B. Magen- Darm-Tumore) und Papilloma-Viren (z.B. Tumoren der weiblichen Geschlechtsorgane).

Besonders gut eignen sich die durch die durch die Ansprüche definierten Verbindungen zur Prophylaxe und zur Behandlung einer der oben aufgeführten Erkrankungen sowie von Hepatitis-C-Virus-Infektionen, gut und bösartigen Tumoren, insbesondere verursacht durch Papilloma-Vieren, und bei der Helicobacter Eradikationstherapie bei Ulcera des Magendarmtraktes.

Zur arzneilichen Anwendung können pharmazeutische Formulierungen, die entweder die isolierten erfindungsgemäßen Substanzen oder lebende oder tote Organismen, die die Substanzen enthalten, alleine oder in Kombination mit weiteren Arzneistoffen eingesetzt werden. Bevorzugt ist die Verwendung in Kombination mit Substanzen, die vom Immunsystem als Fremd- oder Autoantigen erkannt werden.

Beispiele hierfür sind Myelin Basisches Protein (MBP) undweitere Extrakte aus dem Gewebe des Nervensystems, Kollagen des Typs I, II oder III, Thyreoglobulin, Acetylcholinrezeptorprotein, DNS, Inselzell-Extrakte, Humaninsulin, Leberextrakte, Leberzellextrakte, Nebennierenrindenextrakte, Hautextrakte, Herzextrakte, Muskelextrakte, Hautzellextrakte, Extrakte aus Zellen der hämatopoetischen Linie, Augenlinsenproteine, S-Antigene, S-Antigengemische, Magenzellextrakte, Parietalzellextrakte, Intrinsischer Faktor und Darmextrakte.

Bevorzugte Anwendungsformen sind die orale, inhalative, intravenöse, parentale, intracisternale, intravaginale, intraperitoneale, lokale (Puder, Salbe, Tropfen) und rektale Applikation sowie das Auftragen auf die Haut oder Schleimhäute.

Die Erfindung beinhaltet die Applikation eines Inhalationsarzneimittels, das mindestens eine der durch Anspruch 1 definierten Substanzen enthält zur Behandlung von Erkrankungen von Menschen, insbesondere bei Allergien und Atemwegserkrankungen wie Asthma und chronischer Bronchitis.

Als pharmazeutische Zusammensetzungen eignen sich ferner: Tablette, Retard-Tablette, Dragees, Kapseln, Premixe, Pillen, Pellets, Boli, Aerosole, Granulate, Suppositorien, Lösungen, Konzentrate, Suspensionen und Emulsionen, Pasten, Salben, Gele, Cremes, Lotionen, Puder, Infusionen und Sprays. Die pharmazeutischen Formulierungen können einen Bruchteil oder einem Vielfachen einer Einzeldosis entsprechen. Die Dosiereinheiten können z.B. 1,2,3 oder 4 Einzeldosen oder ½, 1/3, oder ¼ einer Einzeldosis enthalten. Eine Einzeldosis enthält vorzugsweise die Menge Wirkstoff, die bei der Applikation verabreicht wird und die gewöhnlich einer ganzen, einer halben oder einem Drittel oder einem Viertel einer Tagesdosis entspricht.

Tabletten, Dragees, Kapseln, Pillen und Granulate können die Wirkstoffe neben den üblichen Trägerstoffen enthalten, wie (a) Füll- und Streckmittel, z. B. Stärken, Milchzucker, Rohr-zucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quartemäre Ammonium-verbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Die Tabletten, Dragees, Kapseln, Pillen und Granulate können mit den üblichen, gegebenen-falls Opakisierungsmittel enthaltenden Überzügen und Hüllen versehen sein und auch so zusammengesetzt sein, daß sie die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Die Wirkstoffe können gegebenenfalls mit einem oder mehreren der oben angegebenen Trägerstoffe auch in mikroverkapselter Form vorliegen.

Suppositorien können neben den Wirkstoffen die üblichen wasserlöslichen oder wasserunlöslichen Trägerstoffe enthalten, z. B. Polyethylenglykole, Fette, z. B. Kakaofett und höhere Ester (z. B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Die notwendigen Mengen der einzelnen Derivate zur Erzielung des gewünschten Effektes unterscheiden sich sehr stark Im allgemeinen hat es sich sowohl in der Human- als auch in der Veterinärmedizin als vorteilhaft erwiesen, den oder die Wirkstoffe der Formel (I) in Gesamtmengen von etwa 0,01 bis etwa 2000 µg je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 0,01 bis etwa 2000 µg. Es kann jedoch erforderlich sein, von den genannten Dosierungen abzuweichen, und zwar in Abhängigkeit von der Art und dem Körpergewicht des zu behandelnden Patienten, der Art und Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Bei der Behandlung von Tieren können die erfindungsgemäß zu verwendenden Verbindungen in den üblichen Konzentrationen und Zubereitungen zusammen mit dem Futter bzw. mit Futterzubereitungen oder mit dem Trinkwasser gegeben werden.

### Beispiel 1

### Aufreinigung von gamma / delta-T-Zell-aktivierenden Verbindungen

Verschiedene gamma / delta-T-Zell-aktivierende Verbindungen wurden aus Corynebacterium ammoniagenes isoliert. 28 kg Zellmasse wurden mit einer Dynax-Mühle in 50 mM Ammoniumformiat-Puffer (pH 8,0) aufgeschlossen. Nach Vorabsorption an einer hydrophoben Polystyrolmatrix wurde der Aufschluß auf einen Anionenaustauscher geladen, der mit einem Stufengradienten (100, 300, 500 mM Ammoniumformiat, pH 8,0) eluiert wurde. Das 300 mM-Eluat wurde über eine C-18-Matrix geleitet und der Durchlauf über einen 3 kDa-Hohlfaserfilter ultrafiltriert. Das Filtrat wurde mit Wasser auf 30 mM Ammoniumformiat verdünnt und erneut auf einen Annionenaustauscher geladen. Die Elution erfolgte mit einem linearen Gradienten von 30 bis 500 mM Ammoniumformiat. Einzelne Fraktionen wurden auf ihre Fähigkeit gamma / delta-T-Zellen zu aktivieren getestet. Zum Teil wurden die aktiven Verbindungen anschließend als Bariumsalze durch Zugabe von 100 mM BaCl₂ und 80 % EtOH gefällt. Die Präzipitate wurden in 20 mM Ammoniumformiat-Puffer (pH 8,0) gelöst und an einem Annionenaustauscher rechromatographiert.

Auf diese Weise können die Verbindungen 1 bis 6 isoliert werden.

### Beispiel 2

### Aktivierung von gamma / delta-T-Zellen durch angereicherte Intermediate des MEP-Wegs

Lymphozyten wurden durch Ficoll-Dichtegradienten-Zentrifugation aus dem peripheren Blut von gesunden Spendern gewonnen. Für jeden Test wurden 2 × 10⁵ der so erhaltenen Zellen in einem Volumen von 0,2 ml RPMI-1640-Medium (Life Technologies) ausgesät, das mit 25 mM HEPES, 2 mM L-Glutamin, 0,025 mg/ml Gentamycin, 100 U/ml humanes Interleukin-2 (IL-2) (alle von Life Technologies), und 10 % humanem AB-Serum (Bayerisches Rotes Kreuz) angereichert war. Die Testfraktionen wurden in verschiedenen Verdünnungen zugesetzt, als Positivkontrolle wurde Isopentenyldiphosphat (IPP) von Sigma in einer Endkonzentration von 10 uM verwendet. Die Inkubation erfolgte bei 37°C und 5 % CO₂ im Brutschrank. Nach 72 Stunden wurden die Zellen geerntet und in einem Durchflußzytometer analysiert. Dabei wurde die Expression des Aktivierungsmarkers CD25 auf der Oberfläche von V gamma 9⁺ T-Zellen gemessen, mit Hilfe der monoklonalen Antikörper CD25-PE (B1.49.9), V gamma 9-FITC (Immu360) und CD3-PC5 (UCHT1) der Firma Beckman-Coulter.

Dabei ergab sich, dass Verbindung 1 etwa 750mal, Verbindung 2 etwa 400mal und die Verbindungen 3, 5, 6 etwa 100mal aktiver sind als IPP.

### Beispiel 3:

Die Synthese erfolgt wie in Schema 1 gezeigt:

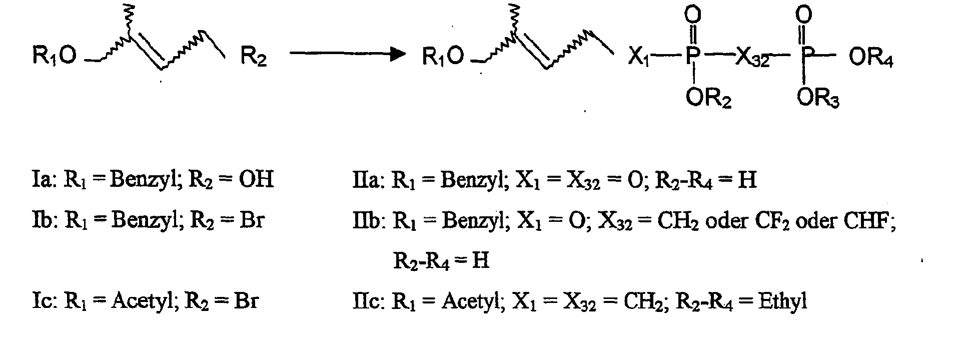

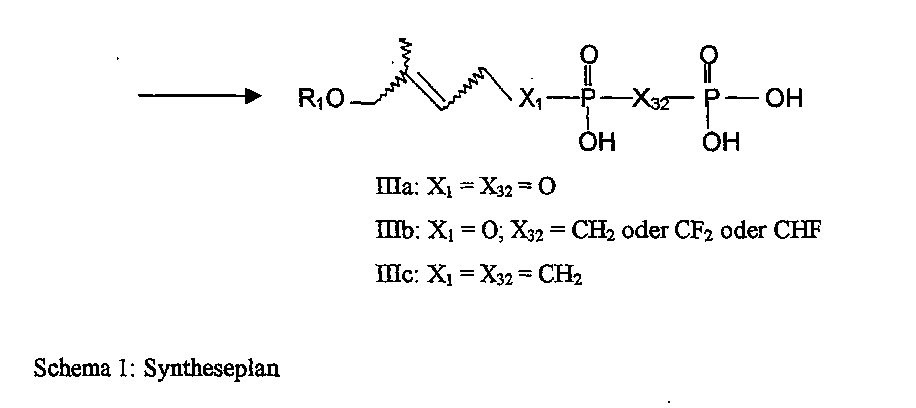

### 1. Herstellung von Verbindungen Ia bis Ic

Verbindung Ia und Ib wurde analog den Vorschriften in: K. Sato, S. Inoue, Y. Takagi, S. Morii, Bull. Chem. Soc. Jpn., 1976, 49(11), 3351-3351 hergestellt.

Die Herstellung von Ic erfolgt analog zu H. Kunio, H. Kazushige, Chem. Pharm. Bull., 1994, 42,4, 786-791.

### 2. Synthesen der Verbindungen IIa bis IIc

Verbindung IIa wurde nach gängigen, dem Fachmann bekannten Verfahren, wie sie z.B. bei B. Woodside, Z. Huang, C.D. Poulter, Org. Synth. 1988, 66, 211-219 beschrieben wurden, ausgehend von Verbindung Ib hergestellt.

Verbindung IIb wurde ausgehend Verbindung Ia hergestellt. Dabei wurde zunächst Ia in das entsprechende Tosylat umgewandelt und anschließend z.B. mit Tris(tetra-n-butylammonium) hydrogenomethylen-diphosphat umgesetzt. Die Synthese wurden analog den in WO00/59916 sowie den darin zitierten Publikationen beschrieben, durchgeführt.

Die Herstellung von Verbindung IIc wiederum erfolgte aus Verbindung Ic. Die Synthesen erfolgten wie bei R.C. McClard und T.S. Fujita, J. Am. Chem. Soc., 1987, 109, 5544-5545 beschrieben.

Verbindung IIc konnte in geringer Ausbeute erhalten werden und wurde gleich hydrolysiert um Verbindung IIIc zu erhalten.

### 3. Synthesen der Verbindungen IIIa bis IIIc

Zur Herstellung von Verbindungen IIIa bis IIIc wurden 500mg der entsprechenden Vorläufer IIa und IIb in je 5ml Methanol gelöst und mit 10 Mol% Hydrierkatalysator versetzt. Anschließend wurde bei Raumtemperatur Wasserstoff eingeleitet, wobei die Aufnahme von Wasserstoff gemessen wurde. Nachdem die entsprechende Menge Wasserstoff aufgenommen worden war, wurde filtriert und das Lösungsmittel abgezogen. Man erhielt das gewünschte Produkt IIIa und IIIb in guter Reinheit. Eine weitere Reinigung kann mit chromatographischen Methoden erreicht werden. Verbindung IIIc wurde aus Verbindung IIc erhalten. Dabei wurden 200 mg von Verbindung IIc in einem ausgeheizten und mit Argon gefluteten Kolben, in absolutem Methylenchlorid (3ml) gelöst und mit 10 eq. Trimethylbromsilan bei 0°C versetzt. Nachdem 1h bei 0°C gerührt wurde, wurde noch weiter 12h bei Raumtemperatur gerührt. Wässrige Aufarbeitung lieferte schließlich das gewünschte Produkt IIIc, welches ionenchromatographisch gereinigt wurde.

Für die Tests zur Aktivierung von gamma/delta-T-Zellen wurden entweder die isomerenreinen oder E/Z-Gemische der Verbindungen eingesetzt.

### Beispiel 4

### Aufreinigung von gamma / delta-T-Zell-aktivierenden Verbindungen

Verschiedene gamma / delta-T-Zell-aktivierende Verbindungen wurden aus Corynebacterium ammoniagenes isoliert. 28 kg Zellmasse wurden mit einer Dynax-Mühle in 50 mM Ammoniumformiat-Puffer (pH 8,0) aufgeschlossen. Nach Vorabsorption an einer hydrophoben Polystyrolmatrix wurde der Aufschluß auf einen Anionenaustauscher geladen, der mit einem Stufengradienten (100, 300, 500 mM Ammoniumformiat, pH 8,0) eluiert wurde. Das 300 mM-Eluat wurde über eine C-18-Matrix geleitet und der Durchlauf über einen 3 kDa-Hohlfaserfilter ultrafiltriert. Das Filtrat wurde mit Wasser auf 30 mM Ammoniumformiat verdünnt und erneut auf einen Annionenaustauscher geladen. Die Elution erfolgte mit einem linearen Gradienten von 30 bis 500 mM Ammoniumformiat. Einzelne Fraktionen wurden auf ihre Fähigkeit gamma / delta-T-Zellen zu aktivieren getestet. Zum Teil wurden die aktiven Verbindungen anschließend als Bariumsalze durch Zugabe von 100 mM BaCl und 80 % EtOH gefällt. Die Präzipitate wurden in 20 mM Ammoniumformiat-Puffer (pH 8,0) gelöst und an einem Annionenaustauscher rechromatographiert. Auf diese Weise konnten die Verbindungen 1 bis 6 und 13 bis 14 isoliert werden.

### Beispiel 5

### Aktivierung von gamma / delta-T-Zellen durch angereicherte Intermediate des MEP-Wegs

Lymphozyten wurden durch Ficoll-Dichtegradienten-Zentrifugation aus dem peripheren Blut von gesunden Spendern gewonnen. Für jeden Test wurden 2 × 10⁵ der so erhaltenen Zellen in einem Volumen von 0,2 ml RPMI-1640-Medium (Life Technologies) ausgesät, das mit 25 mM HEPES, 2 mM L-Glutamin, 0,025 mg/ml Gentamycin, 100 U/ml humanes Interleukin-2 (IL-2) (alle von Life Technologies), und 10 % humanem AB-Serum (Bayerisches Rotes Kreuz) angereichert war. Die Testfraktionen wurden in verschiedenen Verdünnungen zugesetzt, als Positivkontrolle wurde Isopentenyldiphosphat (IPP) von Sigma in einer Endkonzentration von 10 µM verwendet. Die Inkubation erfolgte bei 37°C und 5 % CO₂ im Brutschrank. Nach 72 Stunden wurden die Zellen geerntet und in einem Durchflußzytometer analysiert. Dabei wurde die Expression des Aktivierungsmarkers CD25 auf der Oberfläche von V gamma 9⁺ T-Zellen gemessen, mit Hilfe der monoklonalen Antikörper CD25-PE (B1.49.9), V gamma 9-FITC (Immu360) und CD3-PC5 (UCHT1) der Firma Beckman-Coulter.

Dabei ergab sich, dass Verbindung 9 etwa 10000mal, Verbindung 15, 17 und 19 etwa 500mal, Verbindung 10 etwa 1000mal und Verbindung 12 etwa 50mal aktiver sind als IPP.

## Patentansprüche

1. Verbindungen gemäß Formel (III), 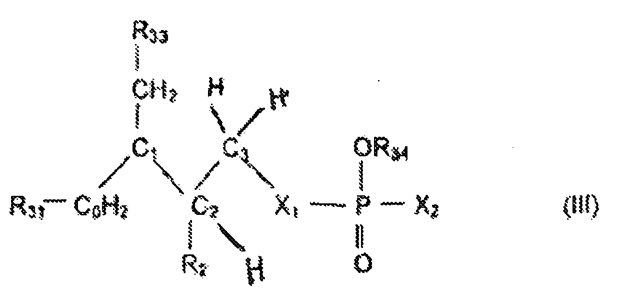 in denen R₃₁ und R₂, die nicht gleichzeitig im Molekül vorhanden sein können, ausgewählt sind aus der Gruppe, die aus OH, substituiertem und unsubstituiertem Phosphat und substituiertem und unsubstituiertem Pyrophosphat besteht, wobei, zwischen C₁ und C₂ eine Doppelbindung gebildet wird, wenn R₃₁ im Molekül vorhanden ist und zwischen C₀ und C₁ eine Doppelbindung gebildet wird, wenn R₂ im Molekül vorhanden ist, R₃₃ ist ausgewählt aus der Gruppe, die aus Wasserstoff, OH, substituiertem und unsubstituiertem Phosphat und substituiertem und unsubstituiertem Pyrophosphat besteht, R₃₄ ist ausgewählt aus der Gruppe die aus Wasserstoff, substituiertem und unsubstituiertem Alkyl mit 1 bis 26 Kohlenstoffatomen, substituiertem und unsubstituiertem Hydroxyalkyl mit 1 bis 26 Kohlenstoffatomen, substituiertem und unsubstituiertem Aryl, substituiertem und unsubstituiertem Aralkyl, substituiertem und unsubstituiertem Alkenyl mit 1 bis 26 Kohlenstoffatomen, substituiertem und unsubstituiertem Alkinyl mit 1 bis 26 Kohlenstoffatomen, substituiertem und unsubstituiertem Cycloalkyl, substituiertem und unsubstituiertem heterocyclischem Rest, substituiertem oder unsubstituiertem Phosphat, einem Silyl, einem Nucleosid, einem Desoxynucleosid, einem Nucleosidmono-, -di- oder triphosphat, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht, X₂ ist entweder -OR₆, wobei R₆ analog R₃₄ definiert ist oder kann 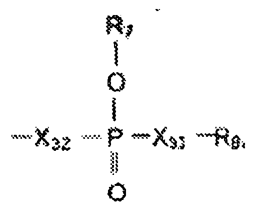 sein, wobei R₇ und R₈ wie R₃₄ definiert sind,
X₁, X₃₂ und X₃₃, die gleich oder verschieden sein können, können Sauerstoff oder eine 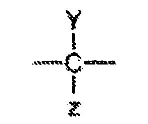 Gruppe sein, wobei Y und Z, die gleich oder verschieden sein können, ausgewählt sind aus der Gruppe, die H, OH, Halogen, Amino, C₁₋₉-Alkoxy, C₁₋₉-Alkylthio, umfasst oder bilden gemeinsam eine Oxogruppe, oder deren pharmaceutischen Salze, Ester der Salze und raümliche Isomer, wobei wenn R₂ ist abwesend, R₃₃ ist H und R₃₁ ist OH, dann das Segment X₁-PO(OR₃₄)-X₂ ist nicht -O-PO(OH)-OPO(OH)₂ oder -O-PO(OH)₂.

2. Verbindungen nach Anspruch 1 **dadurch gekennzeichnet, dass** sie der allgemeinen Formel (IIIA) entsprechen, 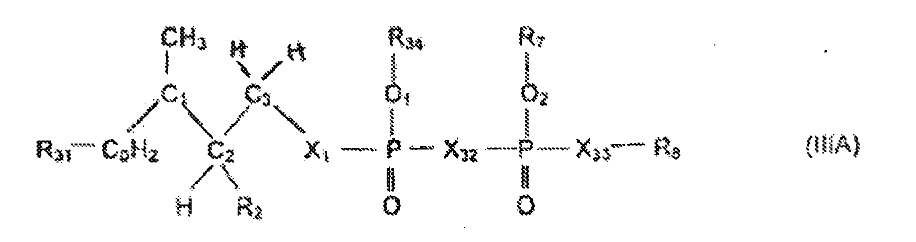 wobei R₃₁ und R₂, die nicht gleichzeitig im Molekül vorhanden sein können, aus der Gruppe ausgewählt sind, die aus OH, substituiertem und unsubstituiertem Phosphat und substituiertem und unsubstituiertem Pyrophosphat besteht, ist R₃₁ im Molekül vorhanden, so wird zwischen C₁ und C₂ eine Doppelbindung gebildet, analog bildet sich zwischen C₀ und C₁ eine Doppelbindung aus, wenn R₂ im Molekül vorhanden ist; R₃₄, R₇, und R₈ die gleich oder verschieden sein können wie in Anspruch 1 definiert sind, X₁, X₃₂ und X₃₃, die gleich oder verschieden sein können, ebenfalls wie in Anspruch 1 definiert sind.

3. Verbindungen nach Anspruch 2 bei denen R₃₁ = OH und C₁ und C₂ durch eine Doppelbindung verbunden sind.

4. Verbindungen gemäß Anspruch 2 bei denen R₂ = OH ist und C₀ und C₁ durch eine Doppelbindung verbunden sind.

5. Verbindungen gemäß Anspruch 1 oder 2 **dadurch gekennzeichnet, dass** entweder R₃₄ oder R₆ oder R₇ oder R₈ ein substituierter oder unsubstituierter Phosphatrest ist.

6. Verbindungen gemäß Anspruch 1 oder 2 **dadurch gekennzeichnet, dass** entweder R₃₁ oder R₂ oder X₂ ein substituierter oder unsubstituierter Phosphatrest ist.

7. Verbindungen gemäß einem der Ansprüche 1 bis 4 oder 6, **dadurch gekennzeichnet, dass** R₃₄, R₆, R₇ und R₈, die gleich oder verschieden sein können, gleich Wasserstoff, einen Kation eines Metalls der 1., 2. oder 3. Hauptgruppe des Periodensystems oder substituiertes oder unsubstituiertes Ammonium sind.

8. Verbindungen gemäß einem der Ansprüche 1 bis 7, **dadurch gekennzeichnet, dass** X₁. und X₃₂ = O sind.

9. Verbindungen gemäß einem der Ansprüche 1 bis 7, **dadurch gekennzeichnet, dass** X₁ = CYZ, X₃₂ = O und X₃₃ = CYZ sind, wobei Y und Z wie in Anspruch 1 definiert sind.

10. Verbindungen gemäß einem der Ansprüche 1 bis 7, **dadurch gekennzeichnet, dass** X₁ = O, X₃₂ = CYZ und X₃₃ = O sind, wobei Y und Z wie in Anspruch 1 definiert sind.

11. Verbindungen gemäß einem der Ansprüche 1 bis 7, **dadurch gekennzeichnet, dass** X₁, X₃₂ und X₃₃, die gleich oder verschieden sein können, ausgewählt sind aus der Gruppe, die aus CH₂, CHF, CHCL, CFCL, CCl₂ oder CF₂ besteht.

12. Verbindungen nach Anspruch 1, ausgewählt aus der folgenden Gruppe :

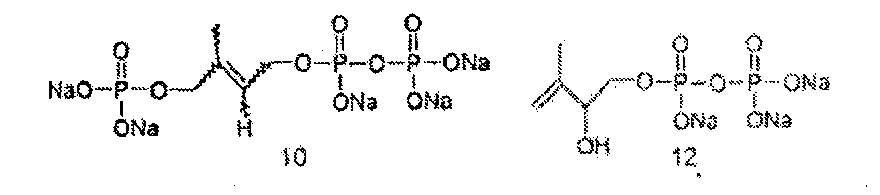

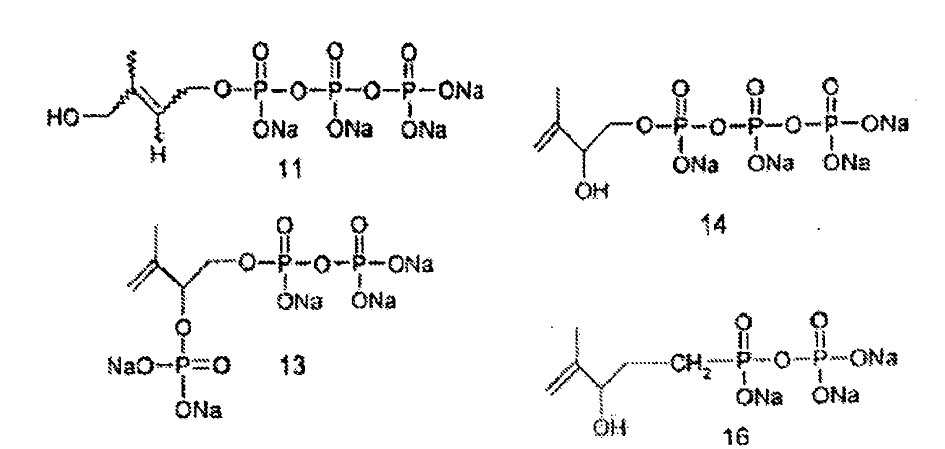

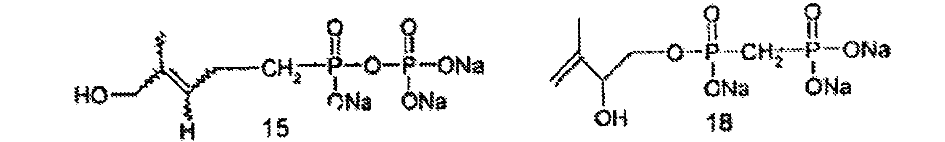

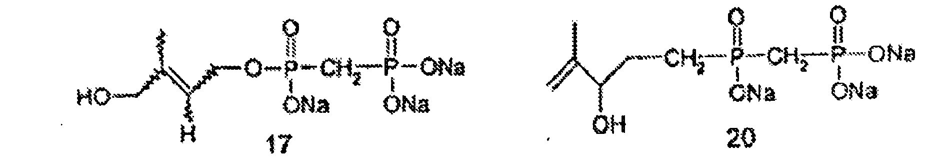

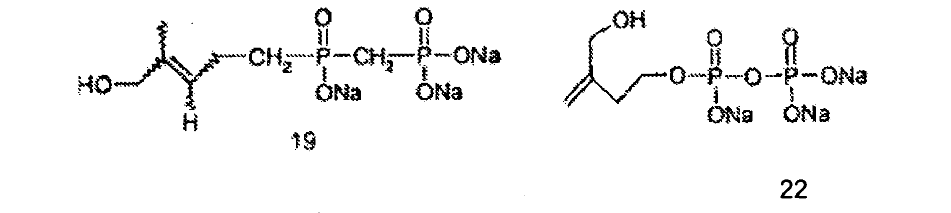

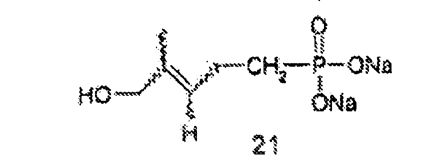

13. Verbindungen nach Anspruch 12, **dadurch gekennzeichnet, dass** der Verbindung ist der Verbindung 15.

14. Verbindungen nach einem Ansprüche 1-13, **dadurch gekennzeichnet, dass** diese eine antibakterielle, antiparasitäre oder antivirale Aktivität aufweisen.

15. Verwendung einer der Verbindungen nach einem der Ansprüche 1-13 zur Herstellung eines Arzneimittels zur Aktivierung von gamma/delta-T-Zellen.

16. Verwendung von Verbindungen nach einem der Ansprüche 1-13 als Substrate oder Produkte in einem Verfahren zur Durchführung von Enzyminhibitionstests und zum Screening von Enzyminhibitoren.

17. Verwendung nach Anspruch 16, **dadurch gekennzeichnet, dass** es sich bei dem Enzym um das LytB-Enzym und / oder GcpE-Enzym handelt.

18. Verwendung von Verbindungen nach einem der Ansprüche 1-13 zur Bestimmung der Aktivierung des LytB-Enzyms und / oder des GcpE-Enzyms.

19. Arzneimittel enthaltend mindestens eine Verbindung gemäß einem der Ansprüche 1-13.

20. Verwendung einer Verbindung gemäß einem der Ansprüche 1-13 zur Herstellung eines Arzneimittels zur Prophylaxe und / oder Behandlung von Erkrankungen von Mensch und Tier.

21. Verwendung nach Anspruch 20 zur Herstellung eines Arzneimittels zur Behandlung von Erkrankungen verursacht durch Viren, Bakterien und Parasiten.

22. Pharmazeutische Zusammensetzung, **gekennzeichnet durch** einen wirksamen Gehalt an zumindest einer der Verbindungen gemäß einem der Ansprüche 1-13 zusammen mit einem pharmazeutisch akzeptablen Träger.

23. Pharmazeutische Zusammensetzung gemäß Anspruch 22, **dadurch gekennzeichnet, dass** mindestens ein weiterer pharmazeutischer Wirkstoff enthalten ist.

24. Verwendung nach Anspruch 20 zur Herstellung eines Arzneimittels zur Verstärkung die Immunabwehr oder zur Induzierung eine immunologische Toleranz gegen Autoantigene und Allergene.

25. Verwendung einer Verbindung gemäß Formel (III), Anspruch 1, oder (IIIA), Anspruch 2, zur Herstellung eines Arzneimittels zur Prophylaxe und/oder Behandlung von Immun-, Autoimmun-, Atemwegserkrankungen und Allergien bei Menschen.

26. Verwendung einer Verbindung gemäß Formel (III), Anspruch 1, oder (IIIA), Anspruch 2, zur Herstellung eines Arzneimittels zur Prophylaxe und/oder Behandlung einer Erkrankung, ausgewählt aus der Gruppe Asthma, Morbus Crohn, Colitis Ulcerosa, Multipler Sklerose, Knochenerkrankungen, insbesondere Osteoporose, und chronischer Bronchitis, sowie rheumathoide Arthritis, Hashimoto-Thyreoiditis, Myasthenia Gravis, Lupus Erythematodes, Diabetes Mellitus, primär-biliäre Zirrhose, aktive chronische Hepatitis, Adrenalinitis/Addison-Krankheit, Polymyositis, Dermatomyositis, Autoimmunhämolytische Anämie, Herzmuskel- und Herzhautentzündungen, Sklerodermie, Uveitis (Phakouveitis, sympathische Opthalmie), Pemphigus Vulgaris, Pemphigoid, Perniziöse Anämie, autoimmune atrophische Gastritis, gut und bösartigen Tumoren, Hepatitis-C-Virus-Infektionen, und Helicobacter Eradikationstherapie bei Ulcera des Magendarmtraktes.

27. Verwendung nach Anspruch 26, zur Herstellung eines Arzneimittels zur Prophylaxe und/oder Behandlung von gut und bösartigen Tumoren and Hepatitis-C-Virus-Infektionen.

28. Verwendung nach Anspruch 27, **dadurch gekennzeichnet, dass** gut und bösartigen Tumoren sind durch Papilloma-Vieren verursacht.

29. Verwendung nach Anspruch 27, zur Herstellung eines Arzneimittels zur Helicobacter Eradikationstherapie bei Ulcera des Magendarmtraktes.

30. Verwendung nach Anspruch 25 oder 26, zur Herstellung eines Inhalationsarzneimittels zur Behandlung von Allergien und Atemwegserkrankungen wie asthma und chronischer Bronchitis.

31. Verwendung nach einem der Ansprüche 20, 21 und 24-29 zur oralen, inhalativen, intravenösen, parentalen, intravaginalen, lokale, oder rektalen Applikation.

32. Verwendung nach Anspruch 31, zur oralen Applikation.

33. Verwendung nach Anspruch 31, **dadurch gekennzeichnet, dass** die pharmazeutischen Formulierung ist ausgewählt aus der Gruppe bestehend aus, Tablette, Retard-Tabelette, Dragees, Kapseln, Premixe, Pillen, Pellets, Boli, Aerosole, Granulate, Suppositorien, Lösungen, Konzentrate, Suspensionen und Emulsionen, Pasten, Salben, Gele, Cremes, Lotionen, Puder, Infusionen und Sprays.

34. Verwendung nach Anspruch 33, **dadurch gekennzeichnet, dass** die pharmazeutische Formulierung ist eine Tablette.

35. Verwendung nach Anspruch 33, **dadurch gekennzeichnet, dass** die pharmazeutische Formulierung ist ein Kapsel.

36. Arzneimittel enthaltend mindestens eine Verbindung gemäß Formel (III), Anspruch 1, oder (III), Anspruch 2, und eine Substanz, die vom Immunsystem als Fremd- oder Autoantigen erkannt werden kann.

37. Arzneimittel nach Anspruch 36, **dadurch gekennzeichnet, dass** die Substanz ist ausgewählt aus der Gruppe bestehend aus, Myelin Basisches Protein (MBP), Extrakte aus dem Gewebe des Nervensystems, Kollagen des Typs I, II oder III, Thyreoglobulin, Acetylchlin-rezeptorprotein, DNS, Inselzell-Extrakte, Humaninsulin, Leberectrakte, Leberzellextrakte, Nebennierenrindenextrakte, Hautextrakte, Herzextrakte, Muskelextrakte, Hautzellextrakte, Extrakte aus Zellen der hämatopoetischen Linie, Augenlinsenproteine, S-Antigene, S-Antigengemische, Magenzellextrakte, Parietalzellextrakte, Intrinsischer Faktor und Darmextrakte.

38. Arzneimittel nach Anspruch 37, **dadurch gekennzeichnet, dass** die Substanz ist Myelin Basisches Protein (MBP).

39. Verwendung einer Verbindung gemäß Formel (III), Anspruch 1, oder (IIIA), Anspruch 2, zur Herstellung eines Arzneimittels zur Prophylaxe und/oder Behandlung einer Erkrankung von Mensch und Tier in Gesamtmengen von etwa 0,01 bis etwa 2000 µg je 24 Stunden.

40. Verwendung nach Anspruch 39, **dadurch gekennzeichnet, dass** eine Einzedlosis enthält die Menge Wirkstoff, die bei der Applikation verabreicht wird and die sewöhnlich einer ganzen, einer halben oder einem Drittel oder einem Viertel einer Tagesdosis entspricht.

41. Verwendung einer Verbindung gemäß einem der Ansprüche 1-13 zur in vitro Aktivierung von gamma/delta-T-Zellen.

## Claims

1. Compounds according to formula (III), 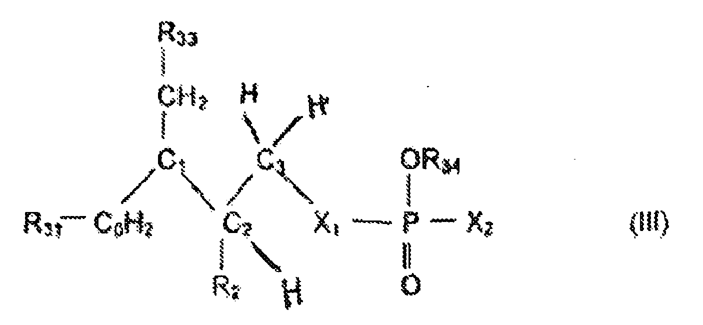 wherein R₃₁ and R₂, which can not be present in the molecule at the same time, are selected from the group consisting of OH, substituted and unsubstituted phosphate and substituted and unsubstituted pyrophosphate, wherein a double bond is formed between C₁ and C₂, if R₃₁ is present in the molecule and wherein a double bond is formed between C₀ and C₁, if R₂ is present in the molecule, R₃₃ is selected from the group consisting of hydrogen, OH, substituted and unsubstituted phosphate and substituted and unsubstituted pyrophosphate, R₃₄ is selected from the group consisting of hydrogen, substituted and unsubstituted alkyl with 1 to 26 carbon atoms, substituted and unsubstituted hydroxyalkyl with 1 to 26 carbon atoms, substituted and unsubstituted aryl, substituted and unsubstituted aralkyl, substituted and unsubstituted alkenyl with 1 to 26 carbon atoms, substituted and unsubstituted alkynyl with 1 to 26 carbon atoms, substituted and unsubstituted cycloalkyl, substituted and unsubstituted heterocyclic moiety, substituted or unsubstituted phosphate, a silyl, a nucleoside, a deoxynucleoside, a nucleoside mono-, di- or triphosphate, a cation of an organic or inorganic base, in particular a metal of the first, second or third main group of the periodic table, ammonium, substituted ammonium and ammonium compounds, which are derived from ethylenediamine or amino acids, X₂ is either -OR₆, wherein R₆ is defined in analogy to R₃₄ or may be 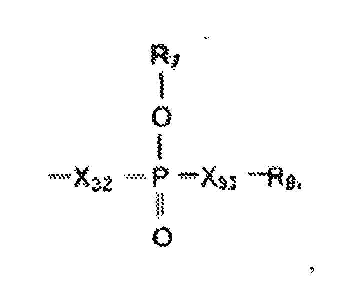 wherein R₇ and R₈ are defined like R₃₄,
X₁, X₃₂ and X₃₃, which may be identical or may differ from each other, may be oxygen or a 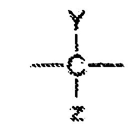 -group,
wherein Y and Z, which may be identical or may differ from each other, are selected from the group comprising H, OH, halogen, amino, C₁₋₉-alkoxy, C₁₋₉-alkylthio, or form together an oxo group, or pharmaceutical salts thereof, esters of said salts and steric isomers, wherein if R₂ is not present, R₃₃ is H and R₃₁ is OH, then the segment X₁-PO(OR₃₄)-X₂ is not -O-PO(OH)-OPO(OH)₂ or -O-PO(OH)₂.

2. Compounds according to claim 1 **characterized in that** they correspond to the general formula (IIIA), 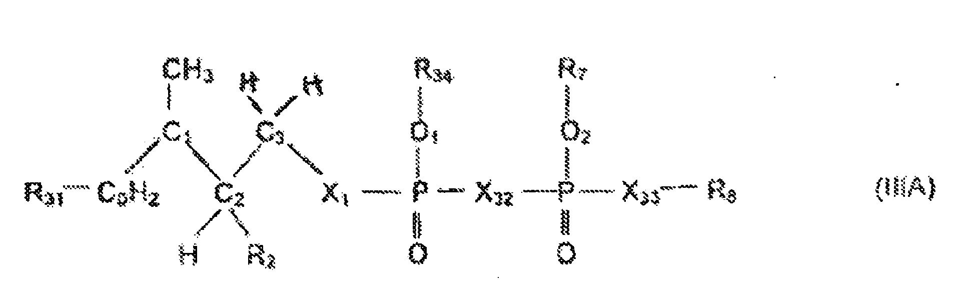 wherein R₃₁ and R₂, which can not be present in the molecule at the same time, are selected from the group consisting of OH, substituted and unsubstituted phosphate and substituted and unsubstituted pyrophosphate, if R₃₁ is present in the molecule a double bond is formed between C₁ and C₂, in an analogous manner a double bond is formed between C₀ and C₁, if R₂ is present in the molecule; R₃₄, R₇ and R₈, which may be identical or may differ from each other, are defined like in claim 1, X₁, X₃₂ and X₃₃, which may be identical or may differ from each other, are defined like in claim 1 as well.

3. Compounds according to claim 2, in which R₃₁ = OH and C₁ and C₂ are linked via a double bond.

4. Compounds according to claim 2, in which R₂ = OH and C₀ and C₁ are linked via a double bond.

5. Compounds according to claim 1 or 2, **characterized in that** either R₃₄ or R₆ or R₇ or R₈ is a substituted or unsubstituted phosphate moiety.

6. Compounds according to claim 1 or 2, **characterized in that** either R₃₁ or R₂ or X₂ is a substituted or unsubstituted phosphate moiety.

7. Compounds according to anyone of claims 1 to 4 or 6, **characterized in that** R₃₄, R₆, R₇ and R₈, which may be identical or may differ from each other, are equal to hydrogen, a cation of a metal of the 1^{st}, 2^{nd} or 3^{rd} main group of the periodic table or substituted or unsubstituted ammonium.

8. Compounds according to anyone of claims 1 to 7, **characterized in that,** that X₁ and X₃₂ are = O.

9. Compounds according to anyone of claims 1 to 7, **characterized in that** X₁ = CYZ, X₃₂ = O and X₃₃ = CYZ, wherein Y and Z are defined like in claim 1.

10. Compounds according to anyone of claims 1 to 7, **characterized in that** X₁ = O, X₃₂ = CYZ and X₃₃ = O, wherein Y and Z are defined like in claim 1.

11. Compounds according to anyone of claims 1 to 7, **characterized in that** X₁, X₃₂ and X₃₃, which may be identical or may differ from each other, are selected from the group consisting of CH₂, CHF, CHCL, CFCL, CCl₂ or CF₂.

12. Compounds according to claim 1 selected from the following group:

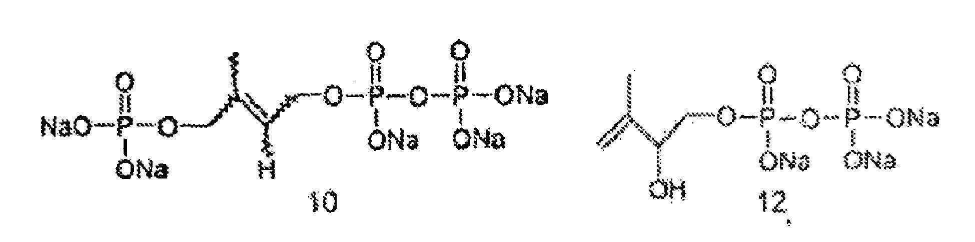

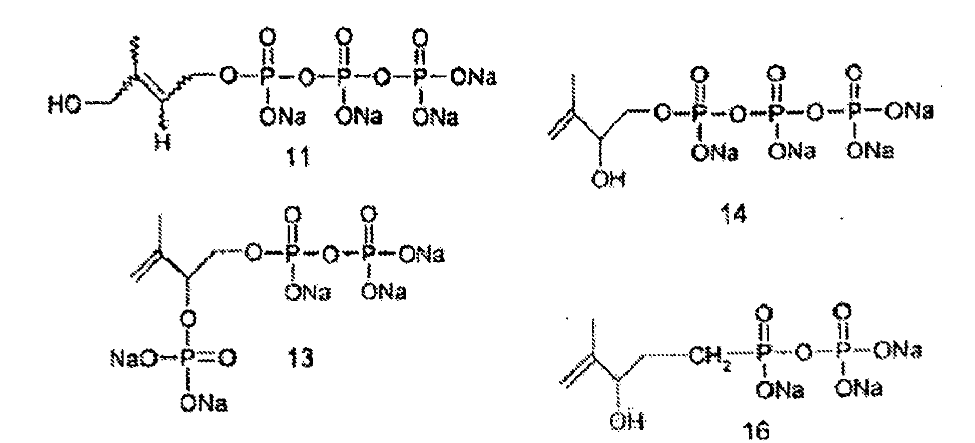

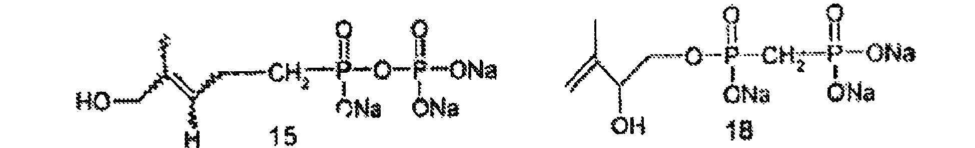

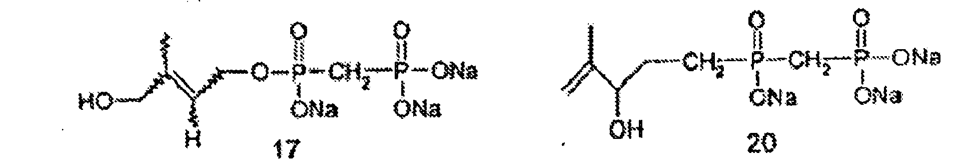

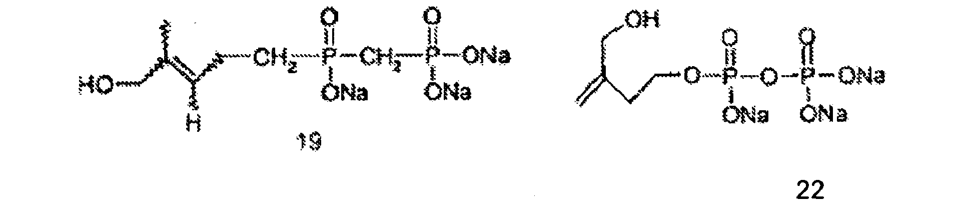

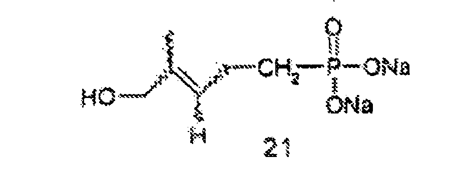

13. Compounds according to claim 12, **characterized in that** the compound is compound 15.

14. Compounds according to anyone of claims 1-13, **characterized in that** they exhibit an antibacterial, antiparasitic or antiviral activity.

15. Use of a compound according to anyone of claims 1-13 for the manufacture of a pharmaceutical for the activation of gamma/delta T-cells.

16. Use of compounds according to anyone of claims 1-13 as substrates or products in a method for performing enzyme inhibition tests and for screening enzyme inhibitors.

17. Use according to claim 16, **characterized in that** the enzyme is the enzyme LytB and/or the enzyme GcpE.

18. Use of compounds according to anyone of claims 1-13 for assessing the activation of the LytB enzyme and/or the GcpE enzyme.

19. Pharmaceutical containing at least one compound according to anyone of claims 1-13.

20. Use of a compound according to anyone claims 1-13 for the manufacture of a pharmaceutical for prophylaxis and/or treatment of diseases of man and animal.

21. Use according to claim 20 for the manufacture of a pharmaceutical for the treatment of diseases caused by viruses, bacteria and parasites.

22. Pharmaceutical composition **characterized by** an effective amount of at least one of the compounds according to anyone of claims 1-13 in combination with a pharmaceutical acceptable carrier.

23. Pharmaceutical composition according to claim 22 **characterized in that** it contains at least a further pharmaceutical agent.

24. Use according to claim 20 for the manufacture of a pharmaceutical for strengthening the immune defence or for inducing an immunological tolerance towards autoantigens and allergens.

25. Use of a compound according to formula (III) of claim 1, or (IIIA) of claim 2, for the manufacture of a pharmaceutical for prophylaxis and/or treatment of immune diseases, autoimmune diseases, diseases of the respiratory tract and allergies of humans.

26. Use of a compound according to formula (III) of claim 1, or (IIIA) of claim 2, for the manufacture of a pharmaceutical for prophylaxis and/or treatment of a disease selected from the group of asthma, Crohn's disease, ulcerative colitis, multiple sclerosis, bone diseases, in particular osteoporosis, and chronic bronchitis, as well as rheumatoid arthritis, Hashimoto's thyroiditis, myasthenia gravis, lupus erythematosus, diabetes mellitus, primary biliary cirrhosis, active chronic hepatitis, adrenalitis/Addison's disease, polymyositis, dermatomyositis, autoimmune hemolytic anemia, inflammation of the heart muscle and endocarditis, scleroderma, uveitis (phacouveitis, sympathetic ophthalmia), pemphigus vulgaris, pemphigoid, pernicious anemia, autoimmune atrophic gastritis, benign and malignant tumours, infections with hepatitis C virus, and eradication therapy of Helicobacter in case of ulcera of the gastrointestinal tract.

27. Use according to claim 26 for the manufacture of a pharmaceutical for prophylaxis and/or treatment of benign and malignant tumours and infections with hepatitis C virus.

28. Use according to claim 27 **characterized in that** the benign and malignant tumours are caused by papilloma viruses.

29. Use according to claim 27 for the manufacture of a pharmaceutical for eradication therapy of Helicobacter in case of ulcera of the gastrointestinal tract.

30. Use according to claim 25 or 26 for the manufacture of a pharmaceutical for inhalation for treatment of allergies and diseases of the respiratory tract like asthma and chronic bronchitis.

31. Use according to anyone of claims 20, 21 and 24-29 for oral, inhalation, intravenous, parental, intravaginal, local or rectal application.

32. Use according to claim 31 for oral application.

33. Use according to claim 31 **characterized in that** the pharmaceutical formulation is selected from the group consisting of tablet, retard tablet, dragées, capsules, premixes, pills, pellets, boluses, aerosols, granulates, suppositories, solutions, concentrates, suspensions and emulsions, pastes, ointments, gels, creams, lotions, powder, infusions and sprays.

34. Use according to claim 33 **characterized in that** the pharmaceutical formulation is a tablet.

35. Use according to claim 33, **characterized in that** the pharmaceutical formulation is a capsule.

36. Pharmaceutical containing at least one compound according to formula (III) of claim 1, or (IIIA) of claim 2, and a substance, which can be recognized by the immune system as foreign or autoantigen.

37. Pharmaceutical according to claim 36 **characterized in that** the substance is selected from the group consisting of myelin basic protein (MBP), extracts of nervous system tissue, type I, II or III collagen, thyreoglobuline, acetylcholine receptor protein, DNA, islet cell extracts, human insulin, liver extracts, liver cell extracts, extracts of the adrenal cortex, skin extracts, heart extracts, muscle extracts, skin cell extracts, extracts of cells of the hematopoetic linage, eye lens proteins, S antigens, S antigen mixtures, stomach cell extracts, parietal cell extracts, intrinsic factor and intestine extracts.

38. Pharmaceutical according to claim 37 **characterized in that** the substance is myelin basic protein (MBP).

39. Use of a compound according to formula (III), claim 1, or (IIIA), claim 2, for the manufacture of a pharmaceutical for prophylaxis and/or treatment of a disease of man or animal in a total amount of about 0.01 to about 2000 µg per 24 hours.

40. Use according to claim 39 **characterized in that** a single dose contains the amount of agent being administered during application and which usually corresponds to a full, half or a third or a fourth of a daily dose.

41. Use of a compound according to anyone of claims 1-13 for the in vitro activation of gamma/delta T-cells.

## Revendications

1. Composés selon la formule (III), 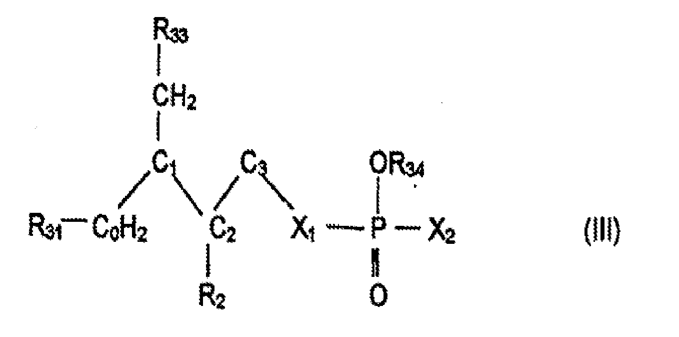 dans lesquels R₃₁ et R₂, qui ne peuvent pas être simultanément présents dans une molécule, sont choisis dans le groupe comprenant un OH, un phosphate substitué et non substitué et un pyrophosphate substitué et non substitué, une double liaison étant formée entre C₁ et C₂ lorsque R₃₁ est présent dans la molécule et une double liaison étant formée entre C₀ et C₁ lorsque R₂ est présent dans la molécule, R₃₃ étant choisi dans le groupe constitué par un hydrogène, un OH, un phosphate substitué et non substitué et un pyrophosphate substitué et non substitué, R₃₄ étant choisi dans le groupe consistant en un hydrogène, un alkyle substitué et non substitué comprenant 1 à 26 atomes de carbone, un hydroxyalkyle substitué et non substitué comprenant 1 à 26 atomes de carbone, un aryle substitué et non substitué, un aralkyle substitué et non substitué, d'alkényle substitué et non substitué comprenant 1 à 26 atomes de carbone, un alkynyle substitué et non substitué comprenant 1 à 26 atomes de carbone, un cycloalkyle substitué et non substitué, un radical hétérocyclique substitué et non substitué, un phosphate substitué et non substitué, un silyle, un nucléoside, un désoxynucléoside, un nucléoside mono-, di- ou triphosphate, un cation d'une base organique et inorganique, notamment un métal du premier, deuxième ou troisième groupe principal du tableau périodique, un ammonium, un ammonium substitué et un composés d'ammonium dérivés d'éthylène diamine ou d'acides aminés, X₂ étant soit -OR₆, R₆ étant défini de manière analogue à R₃₄, soit 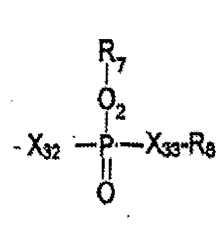 dans lequel R₇ et R₈ étant définis comme R₃₄,
X₁, X₃₂ et X₃₃, qui peuvent être identiques ou différents, peuvent être de l'oxygène ou un groupe 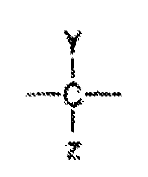 dans lequel Y et Z, qui peuvent être identiques ou différents, sont choisis parmi le groupe consistant en H, OH, halogène, amino, C₁₋₉-alkoxy, C₁₋₉-alkylthio ou définissent ensemble un groupe oxo ou leurs sels pharmaceutiques, des es esters des sels et des isomères spatiaux, toutefois si R₂ est absent, R₃₃ est H et R₃₁ est OH, le segment X₁-PO(OR₃₄)-X₂ n'est pas -O-PO(OH)-OPO(OH)₂ ou -O-PO(OH)₂.

2. Composés selon la revendication 1, **caractérisés en ce qu'**ils correspondent à la formule générale (IIIA), 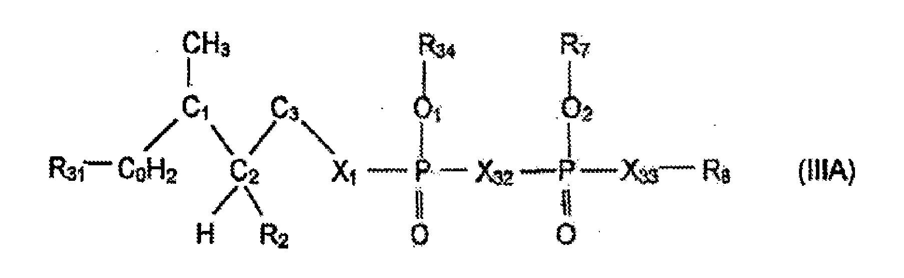 dans laquelle R₃₁ et R₂, qui ne peuvent pas être simultanément présents dans la molécule, sont choisis dans le groupe consistant en OH, un phosphate substitué et non substitué et un pyrophosphate substitué et non substitué, si R₃₁ est présent dans la molécule, une double liaison est formée entre C₁ et C₂, et de manière analogue, il se forme une double liaison entre C₀ et C₁ lorsque R₂ est présent dans la molécule; R₃₄, R₇ et R₈, qui peuvent être identiques ou différents, sont tels que définis dans la revendication 1, X₁, X₃₂ et X₃₃, qui peuvent être identiques ou différents, sont définis également de manière identique que dans la revendication 1.

3. Composés selon la revendication 2, dans lesquels R₃₁ = OH et C₁ et C₂ sont liés l'un à l'autre par une double liaison.

4. Composés selon la revendication 2, dans lesquels R₂ = OH et C₀ et C₁ sont liés l'un à l'autre par une double liaison.

5. Composés selon la revendication 1 ou 2, **caractérisés en ce que** soit R₃₄ soit R₆ soit R₇ soit R₈ est un reste phosphorique substitué ou non substitué.

6. Composés selon la revendication 1 ou 2, **caractérisés en ce que** soit R₃₁ soit R₂ soit X₂ est un reste phosphorique substitué ou non substitué.

7. Composés selon l'une des revendications 1 à 4 ou 6, **caractérisés en ce que** R₃₄, R₆, R₇ et R₈, qui sont identiques ou différents, sont l'hydrogène, un cation d'un métal du 1^{er}, 2^{ème} ou 3^{ème} groupe principal du tableau périodique ou de l'ammonium substitué ou non substitué.

8. Composés selon l'une des revendications 1 à 7, **caractérisés en ce que** X₁ et X₃₂ = 0.

9. Composés selon l'une des revendications 1 à 7, **caractérisés en ce que** X₁ = CYZ, X₃₂ = O et X₃₃ = CYZ, Y et Z étant définis de manière identique que dans la revendication 1.

10. Composés selon l'une des revendications 1 à 7, **caractérisés en ce que** X₁ = O, X₃₂ = CYZ et X₃₃ = O, Y et Z étant définis de manière identique que dans la revendication 1.

11. Composés selon l'une des revendications 1 à 7, **caractérisés en ce que** X₁, X₃₂ et X₃₃, qui peuvent être identiques ou différents, sont choisis dans le groupe consistant en CH₂, CHF, CHCL, CFCL, CC1₂ et CF₂.

12. Composés selon la revendication 1, choisis dans le groupe suivant:

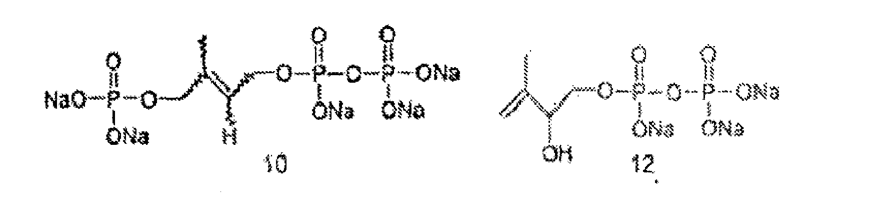

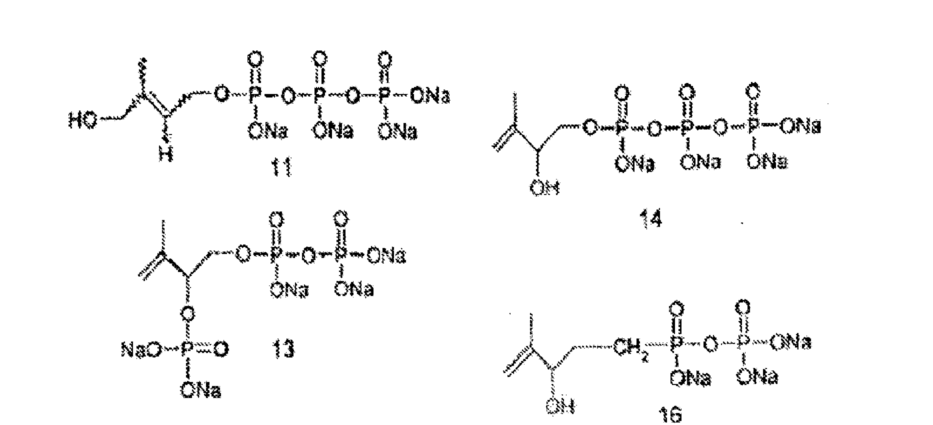

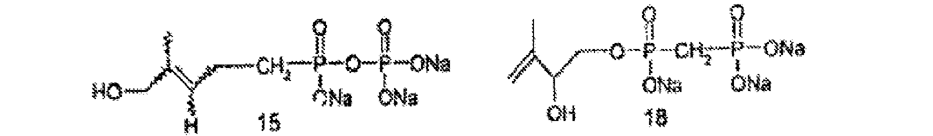

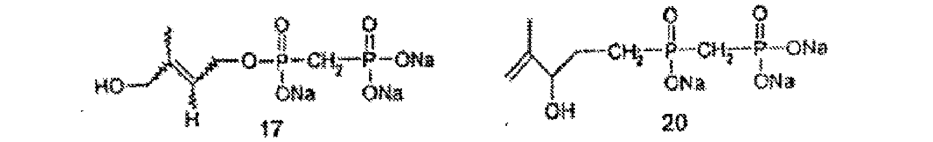

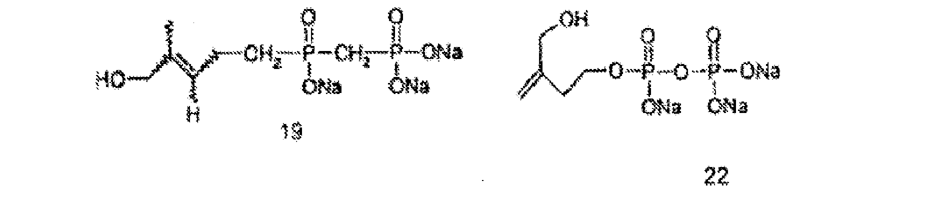

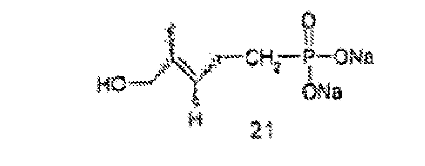

13. Composés selon la revendication 12, **caractérisés en ce que** le composé est le composé 15.

14. Composés selon l'une des revendications 1 à 13, **caractérisés en ce que** ceux-ci ont une activité antibactérienne, antiparasitaire ou antivirale.

15. Utilisation d'un des composés selon l'une des revendications 1 à 13 pour la fabrication d'un médicament pour l'activation de cellules T delta-gamma.

16. Utilisation de composés selon l'une des revendications 1 à 13 comme substrats ou produits dans un procédé pour effectuer des tests d'inhibition enzymatique et de criblage d'inhibiteurs enzymatiques.

17. Utilisation selon la revendication 16, **caractérisée en ce que** l'enzyme est l'enzyme LytB et/ou l'enzyme GcpE.

18. Utilisation de composés selon l'une des revendications 1 à 13 pour la détermination de l'activation de l'enzyme LytB et/ou de l'enzyme GcpE.

19. Médicament contenant au moins un composé selon l'une des revendications 1 à 13.

20. Utilisation d'un composé selon l'une des revendications 1 à 13 pour la fabrication d'un médicament destiné à la prophylaxie et/ou au traitement de maladies humaines et/ou animales.

21. Utilisation selon la revendication 20 pour la fabrication d'un médicament destiné au traitement de maladies provoquées par des virus, bactéries et parasites.

22. Composition pharmaceutique, **caractérisée par** une teneur efficace d'en au moins un composé selon l'une des revendications 1 à 13 en combinaison avec un support acceptable sur le plan pharmaceutique.

23. Composition pharmaceutique selon la revendication 22, **caractérisée en ce qu'**au moins un agent pharmaceutique supplémentaire est contenu.

24. Utilisation selon la revendication 20 pour la fabrication d'un médicament destiné à augmenter les défenses immunitaires ou à induire une tolérance immunologique contre des auto-antigènes et des allergènes.

25. Utilisation d'un composé selon la formule (III) de la revendication 1, ou (IIIA) de la revendication 2, pour la fabrication d'un médicament destiné à la prophylaxie et/ou au traitement de maladies immunologiques, auto-immunes, des voies respiratoires et d'allergies dont peuvent être atteintes les êtres humains.

26. Utilisation d'un composé selon la formule (III) de la revendication 1, ou (IIIA) de la revendication 2, pour la fabrication d'un médicament destiné à la prophylaxie et/au au traitement d'une maladie, choisie dans le groupe consistant en l'asthme, la maladie de Crohn, la colite ulcéreuse, la sclérose multiple, les maladies des os, notamment l'ostéoporose, et la bronchite chronique, ainsi que l'arthrite rhumatoïde, la thyroïdite de Hashimoto, la myasthénie grave, le lupus érythémateux, la diabète, la cirrhose biliaire primaire, l'hépatite chronique active, l'adrénalite/la maladie d'Addison, la polymyosite, la dermatomyosite, l'anémie hémolytique auto-immune, la myocardite et les endocardites, la sclérodermie, l'uvéite (phakouvéite, ophtalmie sympathique), le pemphigus vulgaris, la pemphigoïde, l'anémie pernicieuse, la gastrite atrophique auto-immune, les tumeurs bénignes et malignes, les infections virales de l'hépatite C et la thérapie d'éradication d'Helicobacter en cas d'ulcères du tractus gastro-intestinal.

27. Utilisation selon la revendication 26 pour la fabrication d'un médicament destiné à la prophylaxie et/ou au traitement de tumeurs bénignes et malignes et d'infections virales par l'hépatite C.

28. Utilisation selon la revendication 27, **caractérisée en ce que** les tumeurs bénignes et malignes sont occasionnées par des virus Papilloma.

29. Utilisation selon la revendication 27 pour la fabrication d'un médicament pour la thérapie d'éradication de l'Helicobacter en cas d'ulcères du tractus gastro-intestinal.

30. Utilisation selon la revendication 25 ou 26 pour la fabrication d'un médicament destiné à l'inhalation pour le traitement d'allergies et de maladies des voies respiratoires telles que l'asthme et la bronchite chronique.

31. Utilisation selon l'une des revendications 20, 21 et 24 à 29 pour une administration orale, par inhalation, intraveineuse, parentale, intravaginale, locale ou rectale.

32. Utilisation selon la revendication 31 pour une administration orale.

33. Utilisation selon la revendication 31, **caractérisée en ce que** la formulation pharmaceutique est choisie dans le groupe comprenant les comprimés, comprimés retard, dragées, capsules, prémixes, pilules, pellets, bols, aérosols, granulats, suppositoires, solutions, concentrés, suspensions et émulsions, pâtes, pommades, gels, crèmes, lotions, poudres, infusions et sprays.

34. Utilisation selon la revendication 33, **caractérisée en ce que** la formulation pharmaceutique est un comprimé.

35. Utilisation selon la revendication 33, **caractérisée en ce que** la formulation pharmaceutique est une capsule.

36. Médicament contenant au moins un composé selon la formule (III) de la revendication 1, ou (IIIA) de la revendication 2, et une substance susceptible d'être reconnue comme antigène étranger ou autoantigène par le système immunitaire.

37. Médicament selon la revendication 36, **caractérisé en ce que** la substance est choisie parmi le groupe comprenant la protéine basique de la myéline (MBP), des extraits du tissu neuronal, le collagène du type I, II ou III, la thyréoglobuline, la protéine réceptrice de l'acétylcholine, de l'ADN, des extraits d'ilots de Langerhans, l'insuline humaine, des extraits de foie, des extraits de cellules de foie, des extraits de la corticosurrénale, des extraits de peau, des extraits de coeur, des extraits musculaires, des extraits de cellules de peau, des extraits de cellules de la lignée hématopoïétique, les protéines oculaires, des antigènes S, des mélanges d'antigènes S, des extraits de cellules stomacales, des extraits de cellules pariétales, un facteur intrinsèque et des extraits intestinaux.

38. Médicament selon la revendication 37, **caractérisé en ce que** la substance est de la protéine basique de la myéline (MBP).

39. Utilisation d'un composé selon la formule (III) de la revendication 1, ou (IIIA) de la revendication 2, pour la fabrication d'un médicament destiné à la prophylaxie et/ou au traitement d'une maladie humaine or animale, dans des quantités totales comprises entre 0,01 et 2000 µg environ sur 24 heures.

40. Utilisation selon la revendication 39, **caractérisée en ce qu'**une dose unitaire contient un quantité d'agent destiné à être administré lors de l'application et comprend généralement une dose entière, une demi-dose, ou un tiers ou un quart de la dose journalière.

41. Utilisation d'un composé selon l'une des revendications 1 à 13 pour l'activation in vitro de cellules T delta-gamma.
